# EUROPEAN PATENT APPLICATION

(11) **EP 4 606 426 A1**
(43) Date of publication of application: **27.08.2025**
(21) Application number: 23881805.8
(22) Date of filing: 24.10.2023
(51) Int. Cl.: A61N 5/10

(54) **RADIOTHERAPY SYSTEM AND AUTOMATIC POSITIONING METHOD THEREOF**

(30) Priority: 24.10.2022 CN 202211302897; 20.10.2023 CN 202311367816
(71) Applicant: Neuboron Therapy System Ltd., Xiamen, Fujian 361026 (CN)
(72) Inventor: CHEN, Jiang, Nanjing, Jiangsu 211112 (CN); SHU, Diyun, Nanjing, Jiangsu 211112 (CN); GONG, Qiuping, Nanjing, Jiangsu 211112 (CN)
(74) Representative: Gunzelmann, Rainer
(86) International application number: PCT/CN2023/126076
(87) International publication number: WO 2024/088227

(57) **Abstract**

A radiotherapy system (100) and a positioning method thereof. The radiotherapy system (100) comprises an image acquisition module (1), a placement module (2) used for placing an irradiated body (S), a treatment planning module (4) used for formulating a treatment plan, a radioactive ray generation module (5) used for generating radioactive rays, and a positioning module (6) used for realizing automatic positioning of the placement module (2). The positioning module (6) calculates an irradiation position of the placement module (2) on the basis of the treatment plan generated by the treatment planning module (4), thereby realizing automation and accurate control of positioning of the irradiated body (S), improving the working efficiency, and avoiding misoperation caused by manual participation.

## Description

### TECHNICAL FIELD

The present disclosure relates to the technical field of radiotherapy and computers, and in particular to a radiotherapy system and an automatic positioning method thereof.

### BACKGROUND

With the development of atomics, the radiotherapy such as the cobalt-60, the linear accelerator, and the electron beam has become one of major means to treat cancers. However, the conventional photon or electron therapy is restricted by physical conditions of radioactive rays. Specifically, while tumor cells are killed, a large number of normal tissues on a beam path are damaged. Due to different sensitivities of the tumor cells for the radioactive rays, the conventional radiotherapy is often undesirable to treat radioresistant malignant tumors (such as glioblastoma multiforme and melanoma).

In order to reduce radiation damages to the normal tissues surrounding the tumor, the target therapy in chemotherapy has been employed in the radiotherapy. For the highly radioresistant tumor cells, the radiotherapy with high relative biological effectiveness (RBE), including the proton therapy, the heavy particle therapy, and the neutron capture therapy, has also been developed actively.

For the radiotherapy of the tumor, while the local dose on the tumor is to be maximized, damages to the surrounding normal tissues and complications are to be reduced as much as possible, thereby achieving the higher cure rate of the tumor, and improving the life quality and survival quality of patients. For the sake of a better treatment effect, a position of the patient in the treatment is crucial.

Before the radiotherapy, a treatment scheme is determined by the physician and the physicist through a treatment planning system. Based on medical image information of the patient, including the computed tomography (CT), the magnetic resonance imaging (MRI), and the positron emission tomography-CT (PET-CT), the treatment planning system draws a gross tumor volume (GTV) and a region of interest (ROI) of each important body organ in the medical image, and calculates a radiotherapy dose with a Monte Carlo program, thereby determining the treatment scheme including the position of the patient. Apart from accurate planning on the whole treatment process, during implementation of the radiotherapy, there is a need to position the patient accurately in strict accordance with the treatment scheme determined by the treatment plan. In case of a large positioning error, a central position of the target region of the lesion will change, resulting in an inadequate dose of the target region or an excessive dose of the surrounding normal tissue to obtain an undesirable treatment effect. At present, there lacks an automatic patient positioning system. When the radiotherapy plan is implemented, manual intervention is inevitable, although some auxiliary positioning devices, such as the laser lamp provided on the wall and capable of generating crossed rays, are used.

In order to ensure that the position of the patient in the radiotherapy is the same as the designed position in the treatment plan, improve the patient positioning efficiency, and reduce radiation to the on-site worker, the present disclosure is intended to provide an automatic positioning system, to overcome defects in the prior art, and realize automatic positioning of the patient in the radiotherapy.

### SUMMARY

In view of the above-mentioned technical problems, the present disclosure provides a radiotherapy system and automatic positioning method thereof, to improve the positioning accuracy and the positioning efficiency, and reduce the radiation damage to the on-site worker.

According to an aspect, the present disclosure provides a radiotherapy system, including an image acquisition module configured to acquire a medical image of an irradiated body; a placement module configured to place the radiated body; a treatment planning module configured to formulate a treatment plan; a radioactive ray generation module configured to generate radioactive rays; and a positioning module configured to control positioning of the placement module, where the positioning module is configured to calculate an irradiation position of the placement module on the basis of the treatment plan generated by the treatment planning module.

Further, the positioning module includes markers, at least two laser positioning devices, and a calculation portion configured to calculate the irradiation position of the placement module; and the markers are made of a material capable of being developed in the image.

Further, the placement module includes a placement table configured to place the irradiated body and a mechanical arm configured to move and position the placement table; and the calculation portion is configured to calculate a target position of the mechanical arm and a motion parameter of each joint of the mechanical arm.

Further, the laser positioning devices include an image laser positioning device and an operation laser positioning device that are respectively provided in an image acquisition room and an operation room; the image laser positioning device and the operation laser positioning device each are composed of at least three laser emitters; and a number of the markers is the same as a number of the laser emitters.

Further, the operation room is an irradiation room where irradiation of the radioactive rays is performed.

Further, the radiotherapy system further includes a data management module configured to receive or transmit data with at least one of the image acquisition module, the placement module, the treatment planning module, the radioactive ray generation module, and the positioning module.

According to another aspect, the present disclosure provides an automatic positioning method of a radiotherapy system, where the radiotherapy system is provided with at least an image laser positioning device and an operation laser positioning device; and the automatic positioning method includes the following steps: placing markers at positions where laser beams emitted by the image laser positioning device intersect with an irradiated body; acquiring a medical image of the irradiated body with an imaging device; acquiring a marker coordinate system and a planned beam coordinate system based on a treatment planning module; acquiring a first transformation matrix based on the marker coordinate system and the planned beam coordinate system; fixing the irradiated body on a placement table, and irradiating laser beams emitted by the operation laser positioning device onto the markers correspondingly; and defining a second laser coordinate system of the operation laser positioning device, and determining an irradiation position of the placement table based on the second laser coordinate system and the first transformation matrix.

Further, acquiring a first laser coordinate system of the image laser positioning device based on the treatment planning module specifically includes: importing the medical image into the treatment planning module to obtain the marker coordinate system, and determining the first laser coordinate system based on the marker coordinate system.

Further, acquiring the planned beam coordinate system based on the treatment planning module specifically includes: formulating, by the treatment planning module, a treatment plan based on the medical image, and determining the planned beam coordinate system based on the treatment plan.

Further, determining the irradiation position of the placement table based on the second laser coordinate system and the first transformation matrix includes: determining a beam coordinate system, and determining the irradiation position of the placement table based on the second laser coordinate system, the first transformation matrix and the beam coordinate system.

Further, the radiotherapy further includes a mechanical arm and a flange connected to the mechanical arm and the placement platform; and the automatic positioning method further includes: recording, when the laser beams emitted by the operation laser positioning device are irradiated onto the markers correspondingly, a position of the flange as an initial position; calculating a target position of the flange in combination with the second laser coordinate system and the first transformation matrix; and calculating a motion parameter of the mechanical arm based on the initial position and the target position, where a positioning module controls the placement table based on the motion parameter of the mechanical arm to move to the irradiation position.

In the embodiments of the present disclosure, the positioning module of the radiotherapy system can directly obtain the irradiation position of the placement table in the operation room according to the treatment plan. The present disclosure realizes automation and accurate control of the positioning of the irradiated body, improves the working efficiency, and prevents misoperation caused by manual participation.

### BRIEF DESCRIPTION OF THE DRAWINGS

FIG. 1 is a schematic view of a radiotherapy system according to the present disclosure;
FIG. 2 is a schematic view of a layout of a radiotherapy system according to the present disclosure;
FIG. 3 is a schematic view of a placement module and a positioning module of a radiotherapy system according to the present disclosure; and
FIG. 4 is a schematic view of a beam shaper according to the present disclosure.

In the figures: 100-radiotherapy system, 1-image acquisition module, S-irradiated body, 2-placement module, 21-placement table, 22-mechanical arm, 4-treatment planning module, 5-radioactive ray generation module, 51-neutron generation portion, 511-accelerator, 512-target, 52-beam shaper, 521-reflector, 522-moderator, 523-thermal neutron absorber, 524-radiation shield, 525-beam outlet, 53-collimator, 6-positioning module, 61-marker, 62-operation laser positioning device, 63-calculation portion, 64-control portion, 8-operation room (irradiation room, and preparation room), 81-beam outlet, 9-irradiation room, 91-neutron beam outlet, 92-treatment table, and 93-adjustment portion.

### DETAILED DESCRIPTION OF THE EMBODIMENTS

To make the objectives, technical solutions, and advantages of the present disclosure clearer, the present disclosure is further described in detail below with reference to the drawings and embodiments. It should be understood that the specific embodiments described herein are merely used to explain the present disclosure, rather than to limit the present disclosure.

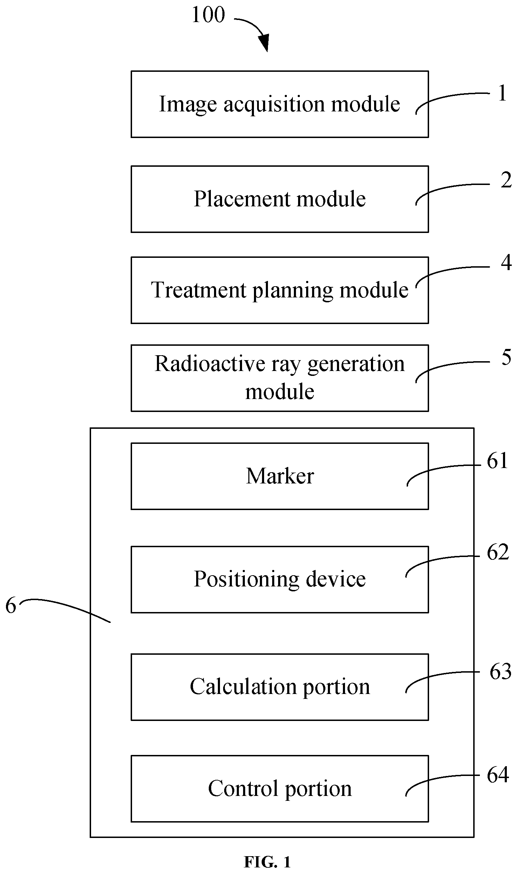

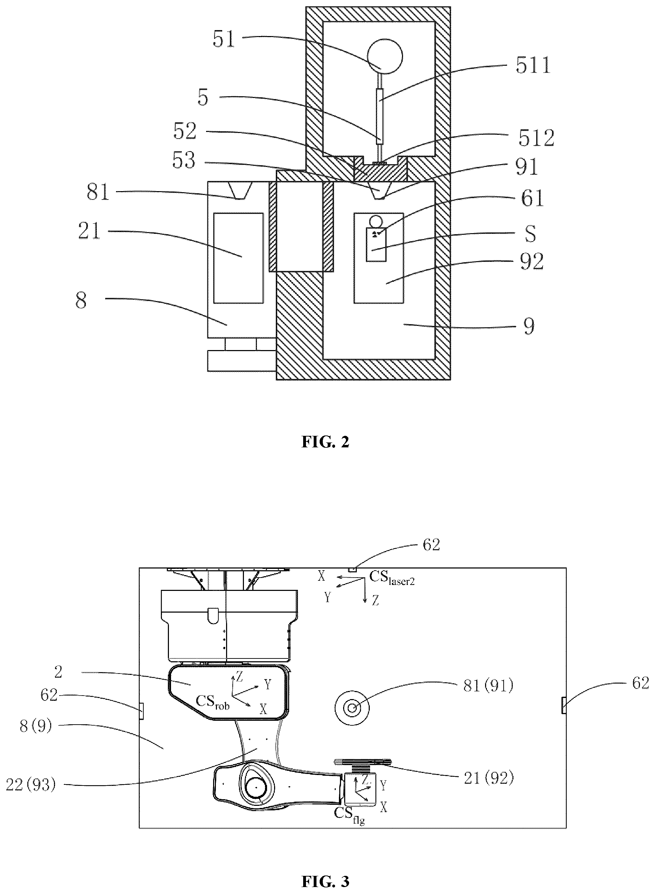

Referring to FIG. 1 and FIG. 2, a radiotherapy system 100 in the embodiment includes an image acquisition module 1 positioned in an image acquisition room (not shown), a placement module 2 positioned in an operation room 8 and configured to place an irradiated body S, a treatment planning module 4 configured to formulate a treatment plan, a radioactive ray generation module 5 configured to generate radioactive rays for irradiation, and a positioning module 6 configured to control positioning of the placement module 2. The image acquisition module 1 is configured to acquire a three-dimensional (3D) medical image of the irradiated body S. The radioactive ray generation module 5 is configured to generate the radioactive rays for treatment. The treatment planning module 4 is configured to perform simulated calculation on a dose according to the 3D medical image of the irradiated body S and data information of a radioactive source generated by the radioactive ray generation module 5 and generate the treatment plan. The treatment plan includes an irradiation position, an irradiation direction, irradiation time, an irradiation dose, etc. The placement module is configured to place the irradiated body S. The positioning module 6 is configured to control a motion of the placement module 2 according to information such as the treatment plan generated by the treatment planning module 4, thereby moving the irradiated body S to the irradiation position.

The positioning module 6 includes markers 61, an image laser positioning device (not shown) provided in the image acquisition room, an operation laser positioning device 62 provided in the operation room 8, a calculation portion 63 configured to calculate the irradiation position of the placement module 2, and a control portion 64 configured to control the motion of the placement module 2 according to a calculated result of the calculation portion 63.

Referring to FIG. 3, the placement module 2 includes a placement table 21 configured to place the irradiated body S, a mechanical arm 22 configured to move and position the placement table 21, and a drive portion configured to drive the mechanical arm 22 to move. The drive portion is preferably a motor. The placement module 2 is provided in the operation room 8. A beam outlet 81 is further formed in the operation room 8. The radioactive rays are emitted from the beam outlet 81 and irradiated onto the irradiated body S for treatment. The calculation portion may be positioned on the placement module, and may also be positioned on other modules in the radiotherapy system, which is not limited herein.

Specifically, the markers 61 are made of a material capable of being developed in the image and easily distinguished from a tissue of the irradiated body S, such as a metal. There are at least three markers 61 that are fixedly provided on the irradiated body S in a non-overlapping manner. In order that the markers 61 can be extracted from the image easily, there are three markers 61 in the embodiment of the present disclosure. The markers 61 are designed as a special shape, such as a triangle, a trapezoid, a parallelogram, and a shape not belonging to the conventional body tissue.

The image laser positioning device and the operation laser positioning device 62 each are composed of a plurality of laser emitters. A number of the laser emitters is the same as a number of the markers 61. Points where laser beams emitted by three laser emitters intersect with the to-be-irradiated body do not coincide with each other, and the three intersection points are respectively aligned to the three markers 61.

In other optional embodiments, the radiotherapy system further includes a data management module configured to receive or transmit data with at least one of the image acquisition module, the placement module, the treatment planning module, the radioactive ray generation module, and the positioning module, thereby realizing information interaction among a plurality of system modules. The calculation portion may also be positioned on the data management module.

An embodiment of the present disclosure provides a positioning method of a radiotherapy system, including the following steps:
Markers are provided on an irradiated body.

An image laser positioning device 62 is provided in an image acquisition room. Three laser emitters of the image laser positioning device 62 are respectively positioned at a left side, a right side and a top side of an origin of an image coordinate system. Laser beams emitted from the three laser emitters intersect with a surface of the irradiated body S. The markers 61 are respectively provided at three positions where the laser beams intersect with the surface of the irradiated body S.

A first laser coordinate system is acquired.

The irradiated body S is scanned with an image acquisition module 1 to obtain an image including at least a lesion of the irradiated body S and the three markers 61. The three markers 61 are extracted from the image, and a marker coordinate system for the three markers 61 is established. The first laser coordinate system CSlaser1 of the image laser positioning device 62 is defined based on the marker coordinate system for the three markers 61. Based on a relative position relationship between the first laser coordinate system CSlaser1 and the image coordinate system CSimage, the first laser coordinate system CSlaser1 under the image coordinate system CSimage is obtained. The relative position relationship may be represented by a transformation matrix.

A planned beam coordinate system is determined.

The 3D medical image of the irradiated body S is imported into a treatment planning module 4. The treatment planning module 4 extracts lesion information from the medical image, and simulates, in combination with a voxel prosthesis tissue model having a tissue type and through a Monte Carlo simulation program, trajectory and energy distributions of radioactive rays in an internal 3D space when the irradiated body S is irradiated by the radioactive rays. Through sampling at different irradiation angles, a dose distribution of the 3D voxel prosthesis tissue model at the different irradiation angles and different irradiation time is calculated. In combination with the dose distribution as well as a dose index (such as a prescribed dose), a dose limit (such as an average dose and a maximum dose), the irradiation angles are selected optimally to generate a treatment plan. The treatment plan including information such as the irradiation position, the irradiation angle, the irradiation time and the irradiation dose is determined.

The planned beam coordinate system CSbeam is constructed based on the irradiation position and the irradiation angle in the treatment plan. A relative position relationship between the planned beam coordinate system CSbeam and the image coordinate system CSimage can be directly obtained from the image, and thus the planned beam coordinate system CSbeam under the image coordinate system CSimage can be obtained. The relative position relationship may be represented by a transformation matrix.

A first transformation matrix T1 between the planned beam coordinate system CSbeam and the first laser coordinate system is acquired.

In combination with the transformation matrix between the planned beam coordinate system CSbeam and the image coordinate system CSimage and the transformation matrix between the first laser coordinate CSlaser1 and the image coordinate system CSimage, a calculation portion 63 calculates the first transformation matrix T1 between the planned beam coordinate system CSbeam and the first laser coordinate system CSlaser1.

Initial positioning is performed on the irradiated body.

The initial positioning is performed on the irradiated body S in an operation room 8. The image laser positioning device 62 is provided in the operation room 8. After the irradiated body S is fixed on a placement table 21, a spatial position of the placement table 21 is adjusted, such that laser beams emitted by the three laser emitters of the operation laser positioning device 62 are irradiated onto the three markers 61 on the irradiated body S correspondingly, thereby completing the initial positioning.

An irradiation position of the placement table is determined.

A mechanical arm coordinate system CSrob is provided in the operation room. An actual beam coordinate system under the mechanical arm coordinate system is defined according to a position of a beam outlet 81 in the operation room 8. For the sake of a better treatment effect, in actual irradiation, the spatial position of the placement table 21 is to be adjusted, such that the relative position relationship between the actual beam coordinate system and the marker coordinate system under the mechanical arm coordinate system is the same as the relative position relationship between the planned beam coordinate system CSbeam and the marker coordinate system in the treatment plan.

Specifically, in the initial positioning, an initial position of a flange connected to the mechanical arm 22 and the placement table 21 in the mechanical arm coordinate system is recorded. By adjusting the position of the flange, the placement table 21 can be driven to move. When the placement table 21 is positioned at the irradiation position, the corresponding position of the flange is defined as a target position. The calculation portion 63 calculates the target position of the flange in combination with a second laser coordinate system CSlaser2, the actual beam coordinate system and the first transformation matrix T1, thereby determining the irradiation position of the placement table.

(7) The mechanical arm is driven to move, thereby driving the placement table to reach the irradiation position.

The calculation portion 63 calculates a motion parameter of the mechanical arm based on the initial position and the target position. Based on the motion parameter of the mechanical arm, a control portion 64 drives the mechanical arm 22 to move, thereby driving the placement table 21 to reach the irradiation position. Specifically, the motion parameter of the mechanical arm is further calculated in combination with a designed parameter of the mechanical arm, which is the conventional art for those skilled in the art, and is not repeated herein.

In other optional implementations, actual controlled positioning in the operation room may also be performed in a flange coordinate system, provided that a position relationship of the marker with a fixed position relative to the beam in the actual positioning is the same as that in the treatment plan to realize accurate treatment.

In an embodiment of the present disclosure, with the image laser positioning device, the operation laser positioning device 62 and the markers 61 on the irradiated body S as media, various modules can cooperate with each other. The irradiation position of the placement table 21 in the operation room 8 is directly obtained according to the treatment plan. This realizes automation and accurate control of the positioning of the irradiated body S, improves the working efficiency, and prevents misoperation caused by manual participation.

Preferably, the radiotherapy system 100 in the present disclosure is a neutron capture therapy system, particularly a boron neutron capture therapy (BNCT) system.

The main principle of the BNCT is as follows: A boron (^{B-}10)-containing pharmaceutical administrated or injected into the irradiated body S are gathered to the tumor cells selectively. With a large capture cross section to thermal neutrons, and through ¹⁰B(n,α)⁷ Li neutron capture and a nuclear fission reaction, the boron(^{B-}10)-containing pharmaceutical generates ⁴He and ⁷Li heavy charged particles. Two heavy charged particles have an average energy of about 2.33 MeV, and have characteristics of high linear energy transfer (LET) and the short range. The two particles have a total range approximately equivalent to a cell size, such that the radiation damage to an organism is limited to a cell level, and a purpose of locally killing the tumor cells on premise of no serious damage to normal tissues can be achieved.

The device for acquiring the 3D medical image may be an imaging device such as a CT device, an MRI device, a PET device and an ultrasound device. In the present disclosure, preferably, the image acquisition module 1 of the CT device is used to acquire medical image data of the irradiated body S through CT. The medical image data of the irradiated body S includes a coordinate matrix and a CT value matrix of a medical image voxel model of a to-be-irradiated portion (a lesion, namely a tumor cell) in a medical image coordinate system. The image acquisition module 1 transmits the medical image data to the data management module.

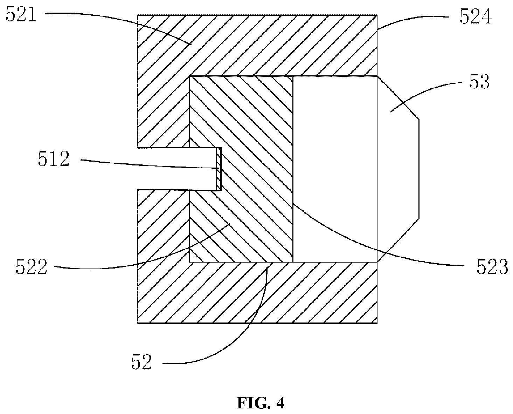

Referring to FIG. 2 and FIG. 4, in the embodiment, the radioactive rays are neutrons. The radioactive ray generation module 5 is a neutron beam irradiation module. The neutron beam irradiation module includes a neutron generation portion 51, a beam shaper 52, and a collimator 53. The neutron generation portion 51 includes an accelerator 511 and a target 512. The accelerator 511 is configured to accelerate charged particles (such as protons and deuterons) to generate a charged particle line such as a proton line. The charged particle line is irradiated onto the target 512 and generates a neutron line (a neutron beam) under an action of the target 512. The target 512 is preferably a metal target 512. An appropriate nuclear reaction is selected according to characteristics such as a desired neutron yield and energy, available energies of the accelerated charged particles, a current, physical and chemical properties of the metal target 512, or the like. Nuclear reactions commonly discussed include ⁷Li(p,n)⁷Be and⁹Be(p,n)⁹B, both of which are endothermic reactions. In the embodiment of the present disclosure, the target 512 is made of lithium. However, as is known to those skilled in the art, the target 512 may also be made of a metal material other than the lithium and beryllium, such as tantalum (Ta) or tungsten (W). The target 512 may be a circular plate, and may also be other solid shapes, and may also be made of a liquid (liquid metal). The accelerator 511 may be a linear accelerator, a cyclotron, a synchrotron, and a synchrocyclotron. In other implementations, the neutron generation portion 51 may be a nuclear reactor without the accelerator 511 and the target 512.

No matter whether a neutron source in the BNCT comes from the nuclear reactor or the nuclear reaction between the accelerated charged particles and the target 512, a mixed radiation field is generated as a matter of fact, that is, the generated beam includes neutrons and photons having energies from low to high. As for the BNCT on deep tumors, except the epithermal neutrons, the higher the content of remaining radioactive rays, the greater the proportion causing non-selective dose deposition in the normal tissue. Therefore, the content of radioactive rays causing the unnecessary dose deposition should be reduced as much as possible. The beam shaper 52 can adjust the quality of a neutron beam generated by the neutron generation device, thereby reducing the unnecessary dose deposition. The collimator 53 is configured to gather the neutron beam, such that the neutron beam has a high targeting ability in the treatment.

The beam shaper 52 includes a reflector 521, a moderator 522, a thermal neutron absorber 523, a radiation shield 524, and a beam outlet 525. The moderator 522 can adjust an energy range (>40 keV) of fast neutrons generated by the neutron generation device to an energy range (0.5 eV to 40 keV) of epithermal neutrons, and reduce a content of thermal neutrons (<0.5 eV) as much as possible. The moderator 522 is made of a material having a large action section with the fast neutrons but a small action section with the epithermal neutrons. As a preferred embodiment, the moderator 522 is made of at least one of D₂O, AlF₃, Fluental^{™}, CaF₂, Li₂CO₃, MgF₂, and Al₂O₃. The reflector 521 surrounds the moderator 522, and reflects neutrons diffused through the moderator 522 back to the neutron beam, thereby improving the utilization rate of the neutrons. The moderator is made of a material with a strong neutron reflectivity. As a preferred embodiment, the reflector 521 is made of at least one of Pb or Ni. On a transmission path of the neutron beam, the thermal neutron absorber 523 is provided behind the moderator 522, configured to absorb thermal neutrons passing through the moderator 522 to reduce the content of the thermal neutrons in the neutron beam, and made of a material having a large action section with the thermal neutrons. As a preferred embodiment, the thermal neutron absorber 523 is made of Li⁻⁶. In other embodiments, because of the Li⁻⁶ in the material of the moderator 522, the thermal neutron absorber 523 may not be provided independently, but the moderator 522 serves as the thermal neutron absorber 523. The radiation shield 524 is configured to shield neutrons and photons leaked from a portion other than the beam outlet 525. A material of the radiation shield 524 includes at least one of a photon shielding material and a neutron shielding material. As a preferred embodiment, the material of the radiation shield 524 includes lead (Pb) as the photon shielding material and polyethylene (PE) as the neutron shielding material.

The collimator 53 is provided behind the beam outlet 525. An epithermal neutron beam from the collimator 53 is irradiated onto the irradiated body S. After passing through a superficial normal tissue of the irradiated body S, the epithermal neutron beam is moderated as thermal neutrons to reach the tumor cells for treatment.

It may be understood that the beam shaper 52 may also be other structures, provided that the epithermal neutron beam can be obtained to meet the treatment requirement. In the present disclosure, the collimator 53 may also not be provided, and the beam from the beam outlet 525 of the beam shaper 52 is directly irradiated onto the irradiated body S. For ease of understanding, when the collimator 53 is not provided, the beam outlet 525 is understood as the beam outlet 81. When the collimator 53 is provided, an outlet of the collimator 53 is understood as the beam outlet 81.

In an embodiment, the operation room 8 is an irradiation room. The irradiated body S positioned in the irradiation room 8 in place is irradiated by the neutron beam for treatment.

In an embodiment, the operation room 8 is a preparation room. The BNCT system further includes an irradiation room 9. Before the irradiated body S is irradiated, preparatory work is made in the preparation room 8. Neutron beam irradiating treatment is performed in the irradiation room 8. Further, a neutron beam outlet 91, a treatment table 92, and an adjustment portion 93 configured to move and position the treatment table 92 are provided in the irradiation room 9. After the placement table 21 is positioned at an irradiation position in the preparation room 8, the positioning module 6 calculates an irradiation position of the treatment table 92 according to information of the irradiation position of the placement table 21. The control portion 64 controls a motion of the adjustment portion 93 according to the irradiation position of the treatment table 92 to move the treatment table 92 to the irradiation position. That is, the automatic positioning method further includes: The calculation portion 63 calculates the irradiation position of the treatment table 92 according to the information of the irradiation position of the placement table 21, and transmits the irradiation position to the control portion 64. The control portion 64 controls the motion of the adjustment portion 93 according to information of the irradiation position of the treatment table 92 to move the treatment table 92 to the irradiation position.

In an embodiment, the placement table 21 and the mechanical arm 22, as well as the treatment table 92 and the adjustment portion 93, are connected together stably in a detachable manner. After the irradiated body S is positioned on the placement table 21 in the preparation room 9, while the relative position relationship between the irradiated body S and the placement table 21 is unchanged, the placement table 21 is detached from the mechanical arm 22. Then, the placement table 21 tied with the irradiated body S is transported to the irradiation room 9 through a device such as a trolley and provided on the adjustment portion 93 in the irradiation room 9. This can restore the relative position relationship between the irradiated body S and the placement table 21 in the irradiation room 9 maximally and simply upon positioning in the preparation room 8, and saves the preparation time in the surgery. In addition, the placement table 21 and the treatment table 92 are a same component, which saves the equipment cost, can reduce a number of times that the irradiated body S is tied to the placement table 21, and improves the working efficiency.

Further, in order to improve the positioning accuracy, first positioning modules and second positioning modules are respectively provided in the preparation room 8 and the irradiation room 9. The first positioning modules and the second positioning modules each are composed of three lasers. Three laser beams from a same positioning module intersect at a point. Specifically, the first positioning modules and the second positioning modules are respectively provided at three sides of the beam outlet 81 and three sides of the neutron beam outlet 91. In the embodiment of the present disclosure, the three lasers of each of the first positioning module and the second positioning module are respectively provided at a left side, a right side and a top side of each of the beam outlet 81 and the neutron beam outlet 91. Specifically, the three lasers of each of the first positioning module and the second positioning module may be provided on a left wall, a right wall and a top ceiling of each of the beam outlet 81 and the neutron beam outlet 91. More specifically, the three lasers of each of the first positioning module and the second positioning module may be provided at a left side, a right side, and a top side at a distance of 10 cm away from each of the beam outlet 81 and the neutron beam outlet 91.

Further, the automatic positioning method further includes the following steps:

A positioning label is formed at a position where three laser beams of the first positioning module intersect with the irradiated body S.

The calculation portion 63 calculates the irradiation position of the treatment table 92 according to the information of the irradiation position of the placement table 21, and transmits the irradiation position to the control portion 64. The control portion 64 controls the motion of the adjustment portion 93 according to information of the irradiation position of the treatment table 92 to move the treatment table 92 to the irradiation position.

Whether an intersection point for three laser beams of the second positioning module in the irradiation room 9 coincides with the positioning label is determined. If yes, the positioning is in place. If no, the spatial position of the treatment table 92 is adjusted, until the intersection point for the three laser beams of the laser positioning module coincides with the positioning label.

Initial positioning is performed in the preparation room 8, and a target position of the treatment table 92 is calculated. A motion parameter of each joint of the adjustment portion 93 is calculated inversely according to the target position of the treatment table 92, thereby directly driving the adjustment portion 93 to move in place. This can save the work time for positioning the irradiated body S before the irradiating treatment in the irradiation room 9, improves the utilization rate of the equipment, and reduces time when an associated worker is exposed in a radioactive environment of the irradiation room 9.

In other implementations, the control portion 64 for controlling the mechanical arm 22 and the adjustment portion 93 according to the motion parameter to move may be not provided in the positioning module 6. The control portion may be an independent control unit, and may also be integrated with other modules of the radiotherapy system.

It should be understood that although the steps in the flowcharts in the above embodiments are shown in sequence as indicated by the arrows, these steps are not necessarily performed in sequence as indicated by the arrows. The execution order of these steps is not strictly limited, and these steps may be executed in other orders, unless clearly described otherwise. Moreover, at least some of the steps in the flowcharts in the above embodiments may include a plurality of steps or stages. The steps or stages are unnecessarily executed at the same time, but may be executed at different times. The execution order of the steps or stages is unnecessarily carried out sequentially, but may be executed alternately with other steps or at least some of the steps or stages of other steps.

The technical characteristics of the above embodiments can be employed in arbitrary combinations. To provide a concise description of these embodiments, all possible combinations of all the technical characteristics of the above embodiments may not be described; however, these combinations of the technical characteristics should be construed as falling within the scope defined by the specification as long as no contradiction occurs.

The above embodiments are merely illustrative of several implementations of the present disclosure, and the description thereof is more specific and detailed, but is not to be construed as a limitation to the patentable scope of the present disclosure. It should be noted that, for a person of ordinary skill in the art, several variations and improvements can be made without departing from the concept of the present application, all of which fall within the protection scope of the present application. Therefore, the protection scope of the present disclosure should be subject to the protection scope defined by the appended claims.

## Claims

1. A radiotherapy system, comprising:
an image acquisition module configured to acquire a medical image of an irradiated body;
a placement module configured to place the irradiated body;
a treatment planning module configured to formulate a treatment plan;
a radioactive ray generation module configured to generate radioactive rays; and
a positioning module configured to control positioning of the placement module, wherein the positioning module is configured to calculate an irradiation position of the placement module on the basis of the treatment plan generated by the treatment planning module.

2. The radiotherapy system according to claim 1, wherein the positioning module comprises markers, at least two laser positioning devices, and a calculation portion configured to calculate the irradiation position of the placement module; and the markers are made of a material capable of being developed in the image.

3. The radiotherapy system according to claim 2, wherein the placement module comprises a placement table configured to place the irradiated body and a mechanical arm configured to move and position the placement table; and the calculation portion is configured to calculate a target position of the mechanical arm and a motion parameter of each joint of the mechanical arm.

4. The radiotherapy system according to claim 3, wherein the laser positioning devices comprise an image laser positioning device and an operation laser positioning device that are respectively provided in an image acquisition room and an operation room; the image laser positioning device and the operation laser positioning device each are composed of at least three laser emitters; and a number of the markers is the same as a number of the laser emitters.

5. The radiotherapy system according to claim 3, wherein the operation room is an irradiation room where irradiation of the radioactive rays is performed.

6. An automatic positioning method of a radiotherapy system, wherein
the radiotherapy system is provided with at least an image laser positioning device and an operation laser positioning device; and
the automatic positioning method comprises the following steps:
placing markers at positions where laser beams emitted by the image laser positioning device intersect with an irradiated body;
acquiring a medical image of the irradiated body with an imaging device;
acquiring a marker coordinate system and a planned beam coordinate system based on a treatment planning module;
acquiring a first transformation matrix based on the marker coordinate system and the planned beam coordinate system;
fixing the irradiated body on a placement table, and irradiating laser beams emitted by the operation laser positioning device onto the markers correspondingly; and
defining a second laser coordinate system of the operation laser positioning device, and determining an irradiation position of the placement table based on the second laser coordinate system and the first transformation matrix.

7. The automatic positioning method according to claim 6, wherein acquiring a first laser coordinate system of the image laser positioning device based on the treatment planning module specifically comprises: importing the medical image into the treatment planning module to obtain the marker coordinate system, and determining the first laser coordinate system based on the marker coordinate system.

8. The automatic positioning method according to claim 6, wherein acquiring the planned beam coordinate system based on the treatment planning module specifically comprises: formulating, by the treatment planning module, a treatment plan based on the medical image, and determining the planned beam coordinate system based on the treatment plan.

9. The automatic positioning method according to claim 6, wherein determining the irradiation position of the placement table based on the second laser coordinate system and the first transformation matrix comprises: determining a beam coordinate system, and determining the irradiation position of the placement table based on the second laser coordinate system, the first transformation matrix and the beam coordinate system.

10. The automatic positioning method according to claim 9, wherein the radiotherapy further comprises a mechanical arm and a flange connected to the mechanical arm and the placement platform; and the automatic positioning method further comprises: recording, when the laser beams emitted by the operation laser positioning device are irradiated onto the markers correspondingly, a position of the flange as an initial position; calculating a target position of the flange in combination with the second laser coordinate system and the first transformation matrix; and calculating a motion parameter of the mechanical arm based on the initial position and the target position, wherein a positioning module controls the placement table based on the motion parameter of the mechanical arm to move to the irradiation position.
